# EUROPEAN PATENT SPECIFICATION

(11) **EP 0 513 183 B1**
(45) Date of publication and mention of the grant of the patent: **13.08.1997**
(21) Application number: 91904201.0
(22) Date of filing: 28.01.1991
(51) Int. Cl.: A61K 7/48, A61K 7/06

(54) **COSMETIC COMPOSITIONS**
KOSMETISCHE ZUSAMMENSETZUNGEN
COMPOSITIONS COSMETIQUES

(30) Priority: 01.02.1990 GB 9002290
(43) Date of publication of application: 19.11.1992
(73) Proprietor: THE PROCTER & GAMBLE COMPANY, Cincinnati, Ohio 45202 (US)
(72) Inventor: DATE, Robert, Francis, Woking Surrey GU21 1QB (GB); STEWART, Karen, Middlesex TW18 4XS (GB); SMITH,Graeme, Douglas, Telfer, Windsor, Berkshire SL4 1HR (GB); CHAMBERS, Gillian, Staines TW18 3AW (GB)
(74) Representative: Brooks, Maxim Courtney
(86) International application number: US9100526
(87) International publication number: WO9111171

(56) References cited:
- WO-A-92/09263
- US-A- 4 837 019
- US-A- 4 863 725
- US-A- 4 956 170
- US-A- 4 965 071

## Description

The present invention relates to skin- and hair-care cosmetic compositions. In particular it relates to cosmetic compositions in the form of substantially oil-free aqueous gels which provide improved skin feel and residue characteristics together with excellent moisturising, emolliency, rub-in and absorption characteristics.

Skin is made up of several layers of cells which coat and protect the keratin and collagen fibrous proteins that form the skeleton of its structure. The outermost of these layers, referred to as the stratum corneum, is known to be composed of 250 Å protein bundles surrounded by 80 Å thick layers. Anionic surfactants and organic solvents typically penetrate the stratum corneum membrane and, by delipidization (i.e. removal of the lipids from the stratum corneum), destroy its integrity. This destruction of the skin surface topography leads to a rough feel and may eventually permit the surfactant or solvent to interact with the keratin, creating irritation.

It is now recognised that maintaining the proper water gradient across the statum corneum is important to its functionality. Most of this water, which is sometimes considered to be the stratum corneum's plasticizer, comes from inside the body. If the humidity is too low, such as in a cold climate, insufficient water remains in the outer layers of the stratum corneum to properly plasticize the tissue; and the skin begins to scale and becomes itchy. Skin permeability is also decreased somewhat when there is inadequate water across the stratum corneum. On the other hand, too much water on the outside of the skin causes the stratum corneum to ultimately absorb three to five times its own weight of bound water. This swells and puckers the skin and results in approximately a two to three fold increase in the permeability of the skin to water and other polar molecules.

Hair consists of many of the same constituents as the stratum corneum. The outermost region of cells forms a rather thick chemically resistant protective coating enclosing the hair fibre which is called the cuticle. The surface of the cuticle is covered with a thin layer called the epicuticle which is thought to contain lipids and protein. The cuticle envelopes the cortex cells which comprise the major part of the fibre mass. Keratinization takes place in the cortex to build stability into the hair structure.

Thus, a need exists for compositions which will assist the stratum corneum and hair cuticle in maintaining their barrier and water retention functions at optimum performance in spite of deleterious interactions which the skin and hair may encounter in washing, work, and recreation.

Conventional cosmetic cream and lotion compositions as described, for example, in Sagarin, Cosmetics Science and Technology, 2nd Edition, Vol.1, Wiley Interscience (1972) and Encyclopaedia of Chemical Technology, Third Edition, Volume 7 are known to provide varying degrees of emolliency, barrier and water-retention (moisturising) benefits. However, they can also suffer serious negatives in terms of skin feel (i.e. they often feel very greasy on the skin) as well as having poor rub-in, absorption and residue characteristics. In the case of hair-care compositions they can also suffer from resoiling negatives.

US-A- 4,837,019 and US-A- 4,863,725 describe skin treatment compositions comprising high levels of polyglyceryl methacrylate lubricant with moisturising components.

The present invention therefore provides skin- and hair-care cosmetic compositions which provide improvements in absorption, residue and skin-feel characteristics without detriment to either short or longer term moisturizing effectiveness or emolliency.

Accordingly, the present invention provides a skin or hair care composition in the form of a substantially oil-free aqueous gel comprising:
(a) from 0.5% to 20% of weight of glycerine;
(b) from 0.1% to 2% by weight of a water-soluble polyglycerylmethacrylate lubricant having a viscosity (10% aqueous solution, 20^{o}C, Brookfield RVT) of less than 4000 mm²s⁻¹⁾ (cps), and a viscosity (neat) in the range of from 700 to 900 mm²s⁻¹ (cps);
(c) from 0.1% to 20% by weight of a hydrophilic gelling agent; and
wherein the level of oil material is less than 1%, preferably less than 0.1% by weight and wherein the polyglyceryl methacrylate lubricant is a hydrate or clathrate formed by the reaction of sodium glycerate with a methacrylic acid polymer.

The compositions of the present invention contain three essential ingredients as well as various optional components as indicated below. All levels and ratios are by weight of total composition, unless otherwise indicated.

A first essential ingredient is glycerine (sometimes known as glycerol or glycerin). Chemically, glycerine is 1,2,3-propanetriol and is a product of commerce. One large source of the material is in the manufacture of soap.

In the present compositions, glycerine is present at a level of from 0.5% to 20%, preferably from 1% to 10%, more preferably from 4% to 8% by weight of composition.

A second essential component is a water soluble polyglycerylmethacrylate lubricant. This generally should have a viscosity (10% aqueous solution, 20^{o}C, Brookfield RVT) of less than 4000 mm²s⁻¹ (cps), preferably less than 1000 mm²s⁻¹ (cps) and more preferably less than 500 mm²s⁻¹ (cps). In addition, the polyglycerylmethacrylate lubricant also has a viscosity (neat) in the range of from 700 to 900 mm²s⁻¹ (cps) (Brookfield RVT, 20^{o}C).

The polyglycerylmethacrylate lubricants which can be used in the compositions of this invention are available under the trademark Lubragel (RTM) from Guardian Chemical Corporation, 230 Marcus Blvd., Hauppage, N.Y. 11787. In general, Lubragels can be described as hydrates or clathrates which are formed by the reaction of sodium glycerate with a methacrylic acid polymer. Thereafter, the hydrate or clathrate is stabilized with a small amount of propylene glycol, followed by controlled hydration of the resulting product. Lubragels are marketed in a number of grades of varying glycerate: polymer ratio and viscosity. Preferred for use herein, however, is so-called Lubragel Oil which has a typical viscosity of 800 mm²s⁻¹ (cps).

In the present compositions, the polyglycerylmethacrylate lubricant is incorporated at a level of from 0.1% to 2%, and preferably from 0.3% to 1% by weight of composition.

The compositions of the invention also contain a hydrophilic gelling agent at a level preferably from 0.1% to 20%, more preferably from 0.2% to 2%, and especially from 0.3% to 1% by weight. The gelling agent preferably has a viscosity (1% aqueous solution, 20^{o}C, Brookfield RVT) of at least 4000 mm²s⁻¹ (cps), more preferably at least 10,000 mm²s⁻¹ (cps), and especially at least 50,000 mm²s⁻¹.

Suitable hydrophilic gelling agents can generally be described as water-soluble or colloidally water-soluble polymers, and include cellulose ethers (e.g. hydroxyethyl cellulose, methyl cellulose, hydroxy propylmethyl cellulose), polyvinylpyrrolidone, polyvinylalcohol, guar gum, hydroxypropyl guar gum and xanthan gum.

Preferred hydrophilic gelling agents herein, however, are acrylic acid/ethyl acrylate copolymers and the carboxyvinyl polymers sold by the B.F. Goodrich Company under the trade mark of Carbopol resins. These resins consist essentially of a colloidally water-soluble polymer of acrylic acid crosslinked with from 0.75% to 2.00% of a crosslinking agent selected from polyallyl sucrose and polyallyl pentaerythritol. Examples include Carbopol 934, Carbopol 940 and Carbopol 950. Carbopol 934 is a water-soluble polymer of acrylic acid crosslinked with 1% of a polyallyl ether of sucrose having an average of about 5.8 allyl groups for each sucrose molecule. A most preferred polymer is Carbopol 950 which has an average molecular weight of 4,000,000.

Neutralizing agents suitable for use in neutralizing acidic group containing hydrophilic gelling agents herein include sodium hydroxide, potassium hydroxide, ammonium hydroxide, monoethanolamine, diethanolamine and triethanolamine.

The compositions of the invention are in aqueous gel form and are preferably formulated so as to have a product viscosity of at least 4,000 and preferably in the range from 4,000 to 300,000 mm²s⁻¹ (cps), more preferably from 20,000 to 200,000 mm²s⁻¹ (cps) amd especially from 80,000 to 150,000 mm²s⁻¹⁾ (cps) (20^{o}C, neat, Brookfield RVT). Preferably the compositions are visually clear. The compositions are also substantially free of oil, i.e. contain less than 1% by weight, and preferably less than 0.1% by weight of materials which are insoluble or which are not colloidally-soluble in the aqueous gel matrix at 10^{o}C. "Colloidally-soluble" herein refers to particles in the usual colloidal size range, typically from 1 to 1000 nm, especially from 1 to 500 nm. In highly preferred embodiments, the compositions are substantially free of materials which are insoluble or not colloidally soluble in distilled water at 20^{o}C. Such materials include many conventional emollient materials such as hydrocarbon oils and waxes, glyceride esters, alkyl esters, alkenyl esters, fatty alcohols, certain fatty alcohol ethers and fatty acid esters of ethoxylated fatty alcohols, sterols extracted from lanolin, lanolin esters, wax esters, beeswax derivatives, vegetable waxes, phospholipids, sterols and amides. The compositions can, however, contain low levels of insoluble ingredients added, for example for visual-effect purposes, e.g. thermochromic liquid crystalline materials such as the microencapsulated cholesteryl esters and chiral nematic (non-sterol) based chemicals such as the (2-methylbutyl)phenyl 4-alkyl(oxy)benzoates available from Hallcrest, Glenview, Illinois 60025, U.S.A.

The compositions of the invention have no need of and are preferably also substantially free of surfactant materials which are conventionally added to cosmetic cream and lotion compositions in order to emulsify a water-insoluble oily phase. Again, "substantially free" means less than 1%, preferably less than 0.1% by weight of the indicated materials. Such emulsifiers include ethoxylated fatty acids, ethoxylated esters, phosphated esters, ethoxylated fatty alcohols, polyoxyethylene fatty ether phosphates, fatty acid amides, acyl lactylates, soaps, etc.

A number of additional water-soluble materials can be added to the compositions of the invention, however. Such materials include the other humectants such as sorbitol, propylene glycol, ethoxylated glucose and hexanetriol; keratolytic agents such as salicylic acid; proteins and polypeptides and derivatives thereof; water-soluble or solubilizable preservatives such as Germall 115, methyl, ethyl, propyl and butyl esters of hydroxybenzoic acid, EDTA, Euxyl(RTM)K400, Bromopol (2-bromo-2-nitropropane-1,3-diol) and phenoxypropanol; anti-bacterials such as Irgasan (RTM) and phenoxyethanol (preferably at levels of from 0.5% to about 5%); soluble or colloidally-soluble moisturising agents such as hylaronic acid, chitin, and starch-grafted sodium polyacrylates such as Sanwet (RTM) IM-1000, IM-1500 and IM-2500 available from Celanese Superabsorbent Materials, Portsmith, VA, USA and described in USA-A-4,076,663; colouring agents; perfumes and perfume solubilizers etc. Water is also present at a level of from 50% to 99.3%, preferably from 80% to 95% by weight of the compositions herein.

The pH of the compositions is preferably from about 4 to about 9, more preferably from about 4.5 to about 7.

The invention is illustrated by the following non-limiting examples.

### Examples I and II

| | I | II |
|---|---|---|
| Glycerine | 5 | 10 |
| Lubragel Oil | 0.5 | 2.0 |
| Carbopol 950 | 0.5 | 1.0 |
| Sodium hydroxide | 0.25 | 0.5 |
| Methyl parabens | 0.2 | 0.2 |
| Euxyl K400 | 0.1 | 0.1 |
| Deionised Water | --------To 100 ------------- | |

The compositions are made by mixing at ambient temperature.

The compositions display improved skin feel and residue characteristics together with excellent moisturizing. emolliency, rub-in and absorption characteristics.

## Claims

1. A skin or hair care composition in the form of a substantially oil-free aqueous gel comprising:
(a) from 0.5% to 20% of weight of glycerine;
(b) from 0.1% to 2% by weight of a water-soluble polyglycerylmethacrylate lubricant having a viscosity (10% aqueous solution, 20°C, Brookfield RVT) of less than 4000 mm²s⁻¹ (cps), and a viscosity (neat) in the range from 700 to 900 mm²s⁻¹ (cps);
(c) from 0.1% to 20% by weight of a hydrophilic gelling agent; and
wherein the level of oil material is less than 1%, preferably less than 0.1% by weight and wherein the polyglycerylmethacrylate lubricant is a hydrate or clathrate formed by the reaction of sodium glycerate with a methacrylic acid polymer.

2. A composition according to claim 1 comprising from 1% to 10%, preferably from 4% to 8% by weight of glycerine.

3. A composition according to claim 1 or 2 comprising from 0.3% to 1% by weight of the polyglycerylmethacrylate lubricant.

4. A composition according to any of claims 1 to 3 having a viscosity (20°C, neat, Brookfield RVT) of from 4000 to 300,000 mm²s⁻¹ (cps).

5. A composition according to any of claims 1 to 4 wherein the gelling agent has a viscosity (1% aqueous solution, 20°C, Brookfield RVT) of at least 4000 mm²s⁻¹ (cps), preferably at least 10,000 mm²s⁻¹ (cps).

6. A composition according to any of claims 1 to 5 wherein the gelling agent is a carboxyvinyl polymer, preferably a colloidally water-soluble polymer of acrylic acid cross-linked with from 0.75% to 2.0% of a cross-linking agent selected from polyallyl sucrose and polyallyl pentaerythritol.

7. A composition according to any of claims 1 to 6 comprising from 0.2% to 2%, preferably from 0.3% to 1% by weight of the gelling agent.

## Patentansprüche

1. Haut- oder Haarpflegezusammensetzung in Form eines im wesentlichen ölfreien wäßrigen Gels, umfassend:
(a) 0,5 bis 20 Gew.-% Glycerin;
(b) 0,1 bis 2 Gew. -% eines wasserlöslichen Polyglycerylmethacrylat-Gleitmittels mit einer Viskosität (10%-ige wäßrige Lösung, 20°C, Brookfield RVT) von weniger als 4.000 mm²s⁻¹ (cps) und einer Viskosität (unverdünnt) im Bereich von 700 bis 900 mm²s⁻¹ (cps);
(c) 0,1 bis 20 Gew.-% eines hydrophilen Geliermittels; und
wobei der Gehalt an Ölmaterial weniger als 1, vorzugsweise weniger als 0,1 Gew.-% beträgt, und wobei das Polyglycerylmethacrylat-Gleitmittel ein Hydrat oder Clathrat ist, das durch die Umsetzung von Natriumglycerat mit einem Methacrylsäurepolymer gebildet worden ist.

2. Zusammensetzung nach Anspruch 1, umfassend 1 bis 10, vorzugsweise 4 bis 8 Gew.-% Glycerin.

3. Zusammensetzung nach Anspruch 1 oder 2, umfassend 0,3 bis 1 Gew.-% des Polyglycerylmethacrylat-Gleitmittels.

4. Zusammensetzung nach mindestens einem der Ansprüche 1 bis 3, mit einer ner Viskosität (20°C, unverdünnt, Brookfield RVT) von 4.000 bis 300.000 mm²s⁻¹ (cps).

5. Zusammensetzung nach mindestens einem der Ansprüche 1 bis 4, wobei das Geliermittel eine Viskosität (1%-ige wäßrige Lösung, 20°C, Brookfield RVT) von mindestens 4.000 mm²s⁻¹ (cps), vorzugsweise mindestens 10.000 mm²s⁻¹ (cps) aufweist.

6. Zusammensetzung nach mindestens einem der Ansprüche 1 bis 5, wobei das Geliermittel ein Carboxyvinylpolymer ist, vorzugsweise eine kolloidal wasserlösliches Polymer von Acrylsäure, die mit 0,75 bis 2,0% eines Vernetzungsmittels, gewählt aus Polyallylsaccharose und Polyallylpentaerythrit, vernetzt ist.

7. Zusammensetzung nach mindestens einem der Ansprüche 1 bis 6, umfassend 0,2 bis 2, vorzugsweise 0,3 bis 1 Gew.-% des Geliermittels.

## Revendications

1. Composition pour les soins de la peau ou des cheveux sous la forme d'un gel aqueux pratiquement exempt d'huile, comprenant :
(a) de 0,5 à 20 % en poids de glycérol;
(b) de 0,1 à 2 % en poids d'un lubrifiant de type poly(méthacrylate de glycéryle) soluble dans l'eau ayant une viscosité (en solution aqueuse à 10 %, à 20°C et sur un viscosimètre Brookfield RVT) inférieure à 4 000 mm²s⁻¹ (cP), et une viscosité (à l'état pur) comprise entre 700 et 900 mm²s⁻¹ (cP);
(c) de 0,1 à 20 % en poids d'un agent gélifiant hydrophile ; et
dans laquelle la quantité de matériau huileux est inférieure à 1 % et de préférence inférieure à 0,1 % en poids, et dans laquelle le lubrifiant de type poly(méthacrylate de glycéryle) est un hydrate ou un clathrate formé par la réaction de glycérate de sodium avec un polymère de l'acide méthacrylique.

2. Composition selon la revendication 1, comprenant de 1 à 10 % et de préférence de 4 à 8% en poids de glycérol.

3. Composition selon la revendication 1 ou 2, comprenant de 0,3 à 1 % en poids du lubrifiant de type poly(méthacrylate de glycéryle).

4. Composition selon l'une quelconque des revendications 1 à 3, ayant une viscosité (à 20°C et à l'état pur sur un viscosimètre Brookfield RVT) comprise entre 4 000 et 300 000 mm²s⁻¹ (cP).

5. Composition selon l'une quelconque des revendications 1 à 4, dans laquelle l'agent gélifiant a une viscosité (en solution aqueuse à 1 %, à 20°C et sur un viscosimètre Brookfield RVT) d'au moins 4 000 mm²s⁻¹ (cP), de préférence d'au moins 10 000 mm²s⁻¹ (cP).

6. Composition selon l'une quelconque des revendications 1 à 5, dans laquelle l'agent gélifiant est un polymère carboxyvinylique, de préférence un polymère soluble dans l'eau à l'état colloïdal d'acide acrylique réticulé avec 0,75 à 2,0 % d'un agent de réticulation choisi parmi le polyallylsaccharose et le polyallylpentaérythritol.

7. Composition selon l'une quelconque des revendications 1 à 6, comprenant de 0,2 à 2 % et de préférence de 0,3 à 1 % en poids de l'agent gélifiant.
